**Europäisches Patentamt**

**European Patent Office**

**Office européen des brevets**

(19)

(11) Numéro de publication: **0 023 892**
**A1**

(12)

# DEMANDE DE BREVET EUROPEEN

(21) Numéro de dépôt: **80830053.7**

(22) Date de dépôt: **21.07.80**

(51) Int. Cl.³: **A 61 L 2/10,** C 02 F 1/32

(30) Priorité: **31.07.79 IT 2477979**

(43) Date de publication de la demande: **11.02.81**
**Bulletin 81/6**

(84) Etats contractants désignés: **AT BE CH DE FR GB LI LU NL SE**

(71) Demandeur: **Vighi, Temistocle, Via Battibue, 14, I-43100 Parma (IT)**

(72) Inventeur: **Vighi, Temistocle, Via Battibue, 14, I-43100 Parma (IT)**

(74) Mandataire: **Paini, Sergio, Dr., V. le Pisa 12, I-20146 Milano (IT)**

(54) **Procédé et dispositif pour la stérilisation des liquides en général avec rayons ultra-violets.**

(57) Le procédé de stérilisation consiste en ce qu'on fait passer le liquide à travers du conduit (2) jusqu'à l'élément (3) en l'exposant à l'action des rayons u.v. germicides émis par le générateur (28) placé dans la gaine (26) de quartz, submergée dans le liquide, qui laisse passer les radiations u.v. pénétrant dans toute la masse liquide circulant entre la surface (16) et la gaine (26).

Le dispositif est constitué par un collecteur (1) d'entrée et par un collecteur (12) de passage du liquide qui communiquent avec un élément (3) de structure tubulaire (4), creuse, allongée avec fond (17); l'élément (3), raccordé aux collecteurs moyennant des tuyaux d'union (2) et (11), forme la structure (4) au centre (18) de laquelle est placée la gaine (26) de quartz. Le circuit tuyaux-élément forme un unique système de vases communicants ouvert en équilibre hydrodynamique avec le liquide à pression constante et en mouvement permanent.

Pour empêcher que le générateur (28) ne subisse le contact direct du liquide, il est placé à l'intérieur d'une gaine (26) submergée dans le liquide et insérée au centre (18) de l'élément (3) qui possède une surface (16) lisse et qui est muni de diaphragmes plats (5) avec orifice (10) et de diaphragmes (6, 7) recourbés avec bague (8) brise-flux.

Un voyant est placé sur un manchon avec un joint et sur la gaine (26) est positionnée la capsule (24) de blocage au moyen d'un autre joint.

ACTORUM AG

## Procédé et dispositif pour la stérilisation des liquides en général avec rayons ultra-violets

La présente invention a pour objet un procédé et son dispo_ sitif relatif pour la stérilisation des liquides en général avec rayons ultra-violets(u.v.); le dispositif est consti_ tué par un collecteur d'entrée et par un collecteur de pas_ sage du liquide communiquant avec un ou plusieurs éléments creux allongés, préférablement tubulaires, de n'importe quelle capacité.

Chaque élément est relié aux collecteurs susdits au moyen de differents conduits d'union, dans lesquelles le premier élément est relié au collecteur d'entrée ou au récipient ou directement au réseau de distribution du liquide en pres_ sion constante et en mouvement permanent dans le circuit des tuyaux ouvert, et dans lequel le dernier élément est jo_ int au conduit de descente du liquide stérilisé.

L'invention se réfère au secteur de la technique de fabri_ cation des dispositifs pour la stérilisation des liquides et particulièrement de l'eau.

En ce qui concerne l'état actuel de la technique dans le domaine d'action de l'invention on connaît des dispositifs qui fonctionnent avec un circuit serpentin tubulaire de verre avec lampes u.v. fixées à l'extérieur ou insérées et submergées dans le liquide avec les désavantages du contact direct des lampes avec le liquide provoquant une diminution de rendement des effets bactéricides optimaux ou une insuf_ fisante pénétration des radiations dans la masse liquide qui circule dans le circuit causée par les courbures du serpentin.

D'autres inconvénients sont: la possibilité de dispersion du courant électrique dans le liquide, la nécessité de vi_ dange du dispositif lorsqu'on doit remplacer les lampes précocement épuisées et, en conséquence, l'interruption du procédé de stérilisation.

La présente invention a pour but une série de remèdes aux inconvenients précédemment cités et résout le problème technico-industriel de créer un procédé et son dispositif de stérilisation avec générateurs de rayons u.v. envelop_ pés dans des gaines spéciales extractibles de quartz optique transparent submergées elles-mêmes dans le liquide qui afflue dans les éléments de stérilisation creux, de forme tubulaire, logeant les gaines avec les générateurs interchangeables susdits, dont les rayons ne sont pas filtrés par les gaines de quartz cités plus haut, laissant inchangées leurs propriétés germicides.

Les avantages obtenus avec l'invention en question con_ sistent en ce que les générateurs ne sont pas baignés par le liquide et donc ils sont soustraits aux effets de la dif_ férence de température du liquide et des générateurs avec rendement total de ceux-ci et durée égale aux heures d'uti_ lisation jusqu'à leur naturel épuisement.

D'autres avantages se relèvent dans le fait d'éviter la vi_ dange de l'installation pour le remplacement des générateurs qui sont insérés dans les gaines et interchangeables à tout moment avec le total rendement des générateurs sans disper_ sion de courant électrique et dans la garantie de stérilisa_ tion complète en vertu des passages forcés du liquide le long des parois tubulaires intérieures des éléments.

Ces éléments sont munis de particuliers diaphragmes de dé_ viation qui provoquent des turbulences dans le liquide et des variations en augmentation et diminution des valeurs de l'énergie cinétique qui a pour conséquence une pro_ longation du temps d'irradiation sur toute la masse li_ quide avec un effet germicide total.

L'invention est exposée ci-après plus en détail à l'aide des planches de dessins ci-jointes qui représentent seule_ ment un exemple de réalisation préféré non limitatif.

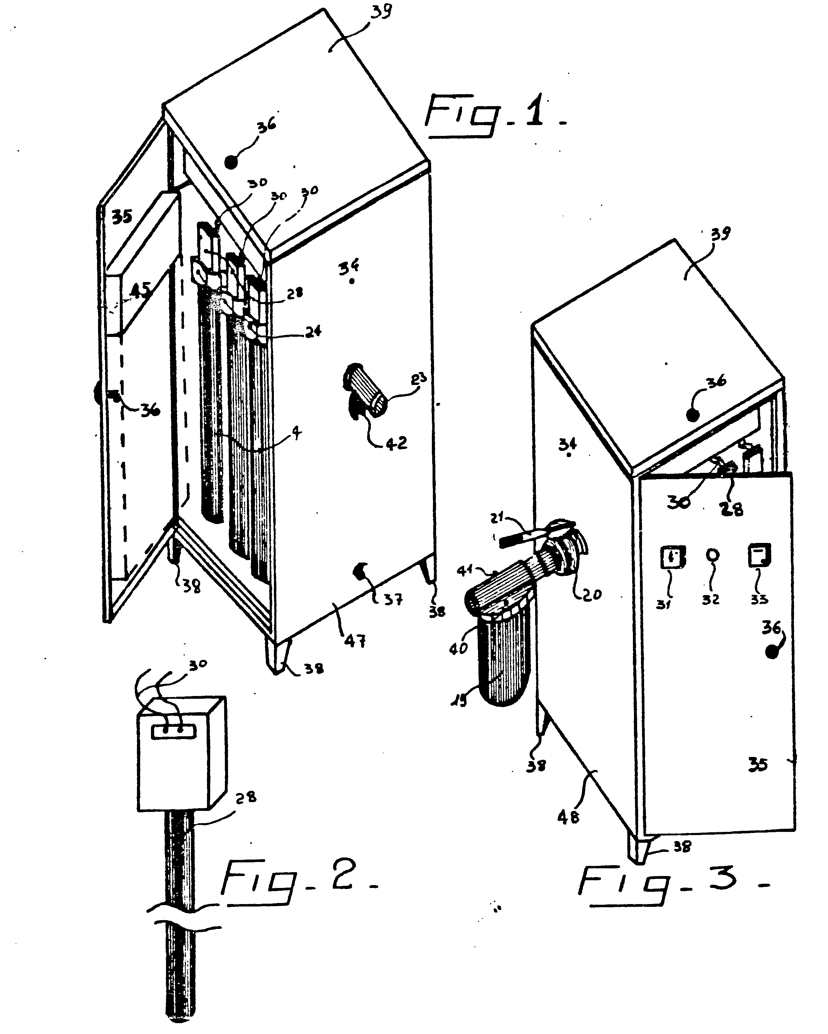

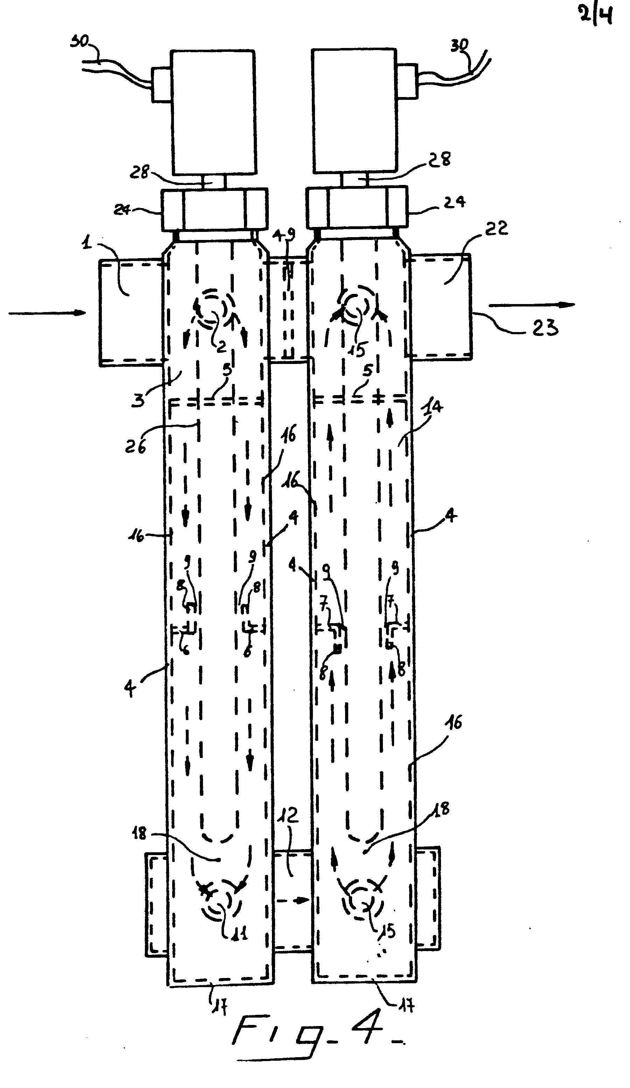

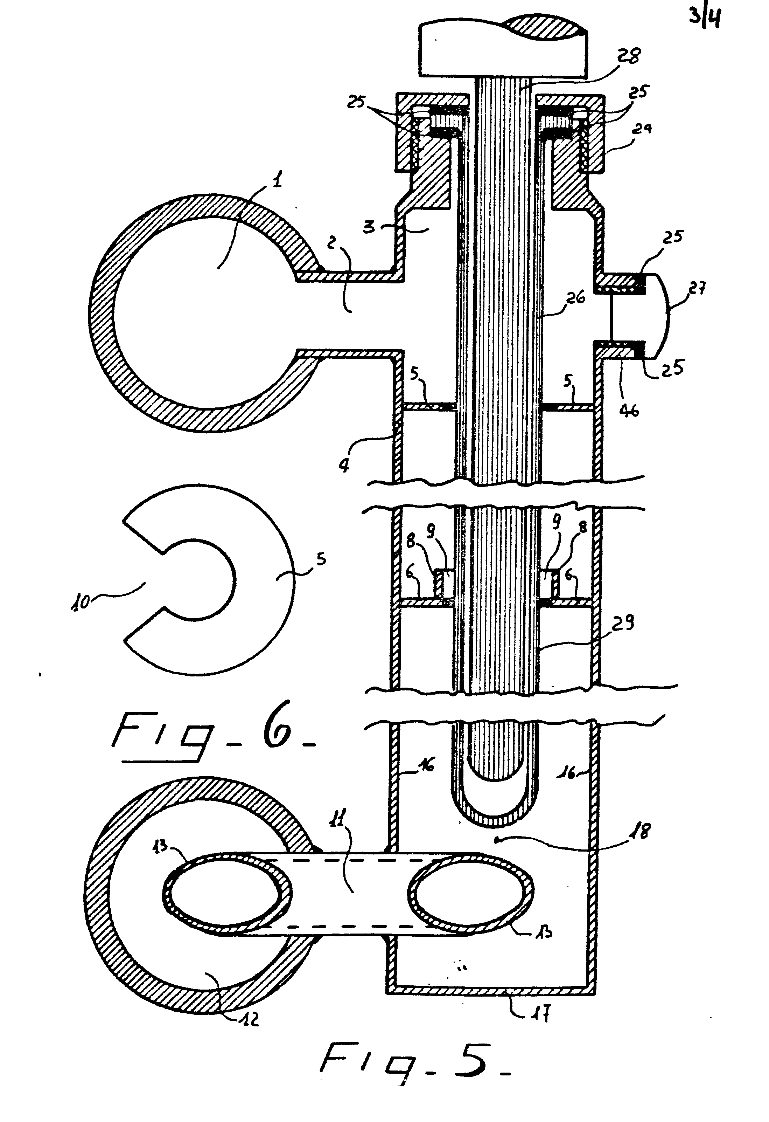

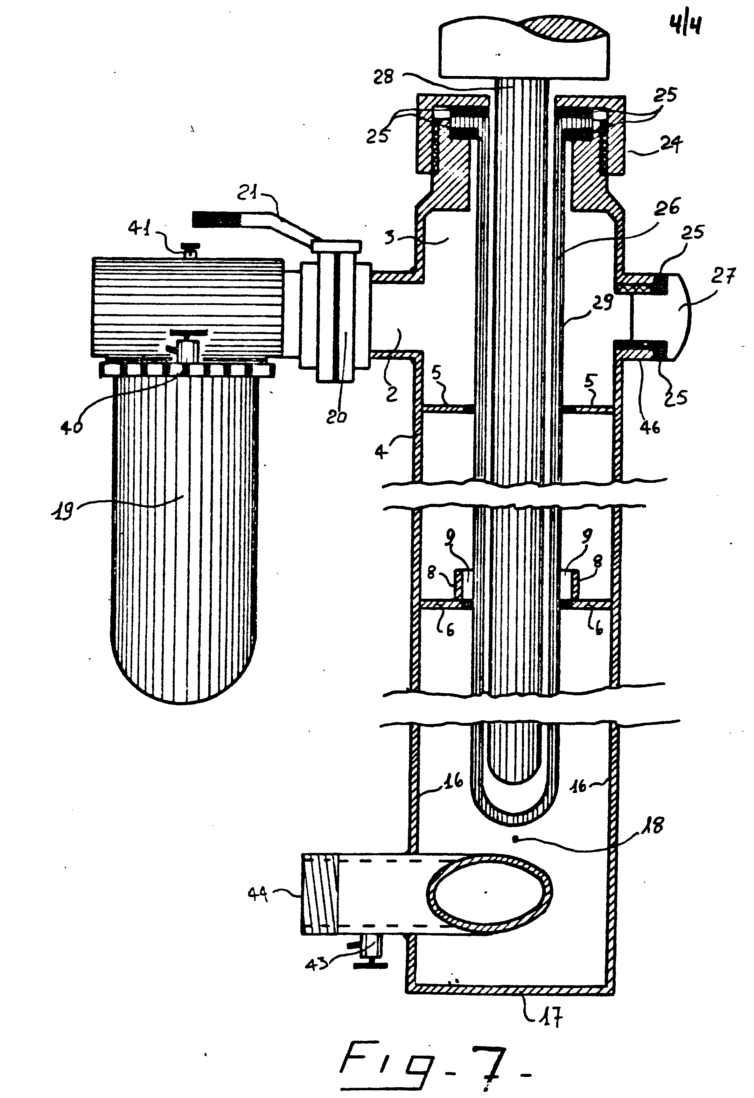

La Fig.1 est une vue en perspective du dispositif montrant la structure en boîtier extérieure et les éléments de sté_ rilisation qui contiennent les générateurs; la Fig.2 est une vue en perspective du générateur avec tête de protec_ tion; la Fig.3 est une vue en perspective du dispositif avec filtre des impuretés en suspension auto-nettoyant et soupape de réglage du débit du liquide en entrée avec le_ vier de commande gradué; la Fig.4 est une vue en élévation des deux éléments, dans lesquels sont insérés les relatifs générateurs, munis de diaphragmes plats et courbés en sens contraire au mouvement du liquide; la Fig.5 est une vue en élévation en coupe d'un élément prévu pour l'accouplement à d'autres en série, montrant le collecteur d'entrée et le collecteur de passage relié à l'élément avec conduit d'u_ nion muni d'orifice elliptique;la Fig.6 est une vue en plan du diaphragme plat spécial avec portion interrompue pour la descente du liquide dans la profondeur de l'élément; la Fig.7 est une vue en élévation en coupe de l'élément prévu pour un dispositif avec élément unique.

Les Figures montrent un dispositif de stérilisation, c'est-à-dire les Figures 1, 2, 3, 4 et 5 se réfèrent au type avec plusieurs éléments, la Fig.7 se réfère au type avec un seul élément et les Fig. 6 et 5 sont communes aux deux types de dispositif.

La constitution de base du type de dispositif avec plu_
sieurs éléments comprend un collecteur 1 d'entrée et un
collecteur 12 de passage du liquide communiquant avec
deux éléments 3 et 14 tubulaires.

L'élément 3 est relié au collecteur 1 moyennant le con_
duit 2 d'union, dans lequel l'élément 3 est relié au col_
lecteur 12 au moyen du conduit 11.

A l'entrée du dispositif est placé le filtre 19 des impuré_
tés en suspension auto-nettoyant, pourvu, vers la descente,
d'une soupape 20 de réglage du débit avec un levier 21.

Le deuxième élément 14, joint au 3, est relié à un conduit
15 de passage du liquide. Les éléments 3 et 14 sont consti_
tués par une structure 4 creuse tubulaire avec parois 16 inté_
rieures lisses dans lesquelles sont logés les générateurs 28,
avec réacteurs connectés au réseau électrique, insérés dans
les gaines 26 de quartz optique tubulaires, avec diamètre
supérieur à celui des générateurs 28, placées au centre 18
de la structure 4 avec fond 17. Les parois 16 possèdent des
diaphragmes plats 5 percés 10 et des diaphragmes 6 recourbés
avec bague 8 brise-flux placés dans l'élément 3 avec courbu_
res 5 vers le haut et dans l'élément 14 avec courbures 7 vers
le bas, tous les deux étant solidaires et transversaux aux
parois 16 et éloignés d'un intervalle 9 des parois 29 exté_
rieures des gaines 26.

L'étranglement de la section des éléments 4 et 14 au moyen des
diaphragmes 5, 6 et 7 cause des tourbillons dans la masse li_
quide avec arrêts forcés à proximité des gaines 26 des généra_
teurs 28 et pour cela un temps majeur d'exposition et pénétra_
tion des irradiations u.v. dans tout le liquide.

L'élément 14 communique avec l'élément 3 et avec le conduit 15 de descente de manière à former un unique système de vases communicants ouvert, en conditions d'equilibre hydrodynamique, avec liquide à pression constante et en mouvement permanent.

Sur chaque gaine extractible est placé une capsule 24 de blocage avec joint d'étanchéité 25.

Le dispositif est pourvu d'un robinet 42 de prélèvement du liquide pour l'analyse bactériologique.

Le générateur 28 est relié au réacteur et au réseau électrique moyennant le fil 30 et est doté d'interrup_ teur 31.

Un voyant 27 est placé sur un manchon 46 et un autre voyant 32 lumineux et un minuteur 33 totalisateur des périodes d'utilisation sont positionnés sur un tableau placé sur le volet 35 de la structure 34 extérieure.

Un couvercle 39 supérieur, qui s'ouvre, permet l'inspec_ tion et l'accès aux générateurs 28 et aux gaines extrac_ tibles. Le couvercle 39 et le volet 35 sont fabriqués de manière hérmétique à simple ou double paroi et sont dotés d'une poignée 36 avec serrure.

La structure 34 présente les parois latérales 47 et 48 et se pose sur le sol avec les pieds 38. Un robinet 37 de vidange du dispositif est placé sur le collecteur 12 et sur la paroi 47. Le filtre 19 est doté d'un robinet 40 de vidange des sédimentations filtrées et d'un robinet 41 pour l'échappement de l'air du filtre 19.

La constitution de base du type de dispositif avec un seul élément comprend les mêmes principes que celui avec plusieurs éléments, avec les particularités suivantes : l'entrée du liquide qui vient du filtre 19 et de la sou_ pape 20 arrive directement dans le conduit 2 d'entrée.

La sortie du liquide stérilisé arrive dans le conduit 44 de descente pourvu de robinet 43 de prélèvement et vi_ dange du dispositif.

On esplique ci dessous brièvement le fonctionnement du dispositif.

L'opérateur doit fermer le circuit électrique à l'aide de l'interrupteur 31 qui allume les générateurs 28.

Le liquide arrive au filtre 19 et à travers la soupape 20 arrive au collecteur 1 d'entrée et ensuite au premier élément 3. Puis il descend à travers l'orifice 10 du diaphragme 5 et se met en contact avec les parois 29 de la gaine 26 par l'effet de son rapide mouvement obtenu avec les diaphragmes 5, 6 avec bague 8 et courbures 6 et 7 qui l'obligent à s'écouler dans l'intervalle 9 avec variations en augmentation et diminution de l'énergie cinétique. Puis le liquide arrive au collecteur 12 avec conduit 11 intérieur et orifice 13 elliptique qui mélangent de nouveau toute la masse liquide qui ensuite s'écoule au moyen du conduit 15 d'union dans l'élément 14.

Le liquide passe encore, comme il est montré dans les Fig. 4, 5 et 6, à travers l'intervalle 7 et donc à travers l'orifice 10 du diaphragme 5 au conduit d'écoulement 22 avec passage préalable dans le conduit 15 avec direction

7

0023892

de la flèche 23 pour être utilisé ou emmagasiné en réci_
pients stérilisés.

Le dispositif peut s'adapter à plusieurs variations construc_
tives qui ne sortent pas de l'idée de solution de l'invention.

On donne ici quelques exemples. Au lieu des diaphragmes 5,
6 et 7 des étranglements et des dilatations peuvent être
réalisés, de manière alternée, intérieurement aux éléments
3 et 14 pour obtenir des mouvements turbulents dans le li_
quide.  En outre le dispositif peut être fabriqué avec élé_
ments jumélés avec deux ou plusieurs couples d'éléments
reliés entre eux par des conduits d'union séparés de parois
49.  Dans le premier couple d'éléments le liquide descend
et dans le deuxième couple il monte en augmentant, par l'ef_
fet du double passage, le temps d'exposition du liquide aux
rayons u.v.

Les éléments 3 et 14 de stérilisation pourront être placés,
par rapport au collecteur 1, sur un côté ou sur les deux
côtés ou de manière alternée.  Pour des dispositifs avec
débit supérieur à 15.000 litres/heure, la structure 34
pourra avoir des formes et des encombrements différents
s'adaptant aux nécessités; en outre sont prévues: la fabri_
cation avec double paroi des côtés extérieurs 47 et 48, du
couvercle 39 et du volet 35 de la structure 34, ainsi que
le placement du tableau des instruments et de chaque appa_
reil électrique sur les côtés 47 et 48 avec plusieurs vo_
lets pour l'inspection à l'intérieur, non montrés dans les
Figures.  Il a été prévu une électro-vanne qui pourvoit à
fermer la descente du liquide en cas de panne du dispositif.

Le dispositif relatif à cette invention est en particulier adapté à détruire tous les types de bactéries, même les plus résistantes, comme par exemple, dans le cas de sté_ rilisation de l'eau, le streptocoque fécal, le coliforme, l'escherichia coli, le clostride sultito-réducteur et d'autres encore. Ces bactéries originales ou dérivées sont complètement détruites par les rayons u.v. parce que ceux-ci pénètrent rationnellement en vertu du nouveau procédé de sterilisation avec le maximum d'énergie dans toute la masse du liquide dans le dispositif.

Naturellement, le principe de l'invention ne variant pas, les formes d'exécution et pratiquement tous les détails de réalisation pourront largement varier, en rapport à ce qui a été expliqué et montré, sans sortir du cadre de la présente invention.

## Revendications

1. Procédé pour la stérilisation des liquides en général, caractérisé en ce qu'on fait passer le liquide à tra_ vers le conduit (2) d'union jusqu'à un ou plusieurs éléments (3) et (14) creux allongés en pression constan_ te et en mouvement permanent dans un système d'éléments (3) et (14) et conduits (2) ouvert en équilibre hydro_ dynamique en l'exposant à l'action totale des radiations u.v. germicides émises par les générateurs (28) placés à l'intérieur de gaines (26) autour desquelles circule le liquide qui est irradié en toute sa masse moyennant un ou plusieurs passages forcés le long des générateurs (28) susdits, selon le nombre des éléments (3) et 14) au moyen d'une série de passages et de variations en augmentation et diminution des valeurs de l'énergie cinétique du liqui_ de, cela à l'aide de particuliers diaphragmes plats (5) et recourbés (6) avec courbures (6) vers le haut et cour_ bures (7) vers le bas placés le long de la surface (16) intérieure des éléments (3) et (14) pour prolonger le temps d'exposition aux rayons u.v. germicides jusqu'à la destruction totale de toutes les bactéries contenues dans ce liquide.

2. Dispositif pour la stérilisation des liquides en géné_ ral avec rayons u.v., constitué par un collecteur (1) d'entrée et par un collecteur (12) de passage du liquide communiquant avec un ou plusieurs éléments (3) et (14) tubulaires creux de n'importe quelle capacité, chaque élément (3) et (14) étant raccordé aux collecteurs (1) et (12) moyennant des tuyaux (2) et (11) d'union, dans lequel le premier ou unique élément (3) est raccordé au collecteur (1) ou au récipient ou directement au réseau du liquide en pression constante et en mouvement perma_ nent en distribution, avec dispositif (19) de filtrage des impuretés en suspension auto-nettoyant placé à l'en_ trée de la conduite du dispositif, et dans lequel le dernier ou unique élément (14) est raccordé à un tuyau (15) de descente du liquide stérilisé, caractérisé en ce que chaque élément (3) et (14) de stérilisation est constitué par une structure (4) creuse allongés avec surface (16) lisse à l'intérieur dans laquelle est logé un générateur (28) de rayons u.v. placé dans une gaine (26) allongée de quartz optique transparent de dimension supérieure au générateur (28) placée au centre (18) de la structure (4) et caractérisé en ce que la surface lis_ se(16) intérieure possède un diaphragme (15) supérieur plat avec orifice (10) et un ou plusieurs diaphragmes (6) et (7) munies d'une bague (8) fixés solidairement et transversalement à la surface (16) avec un interval_ le(9) de la surface (29) extérieure de la gaine (26), et caractérisé ultérieurement en ce que chaque élément (3), (14) ou l'unique (14) est communicant avec le pré_ cédent (3) ou avec le conduit (2) d'alimentation et avec l'élément (14) suivant ou avec le conduit (15) de de_ scente de nature à former un unique système de vases communicants à circuit ouvert en conditions d'équili_ bre hydrodynamique avec liquide à pression constante et en mouvement permanent.

3. Dispositif pour la stérilisation des liquides en géné_ ral, selon la revendication 2, caractérisé en ce que chaque générateur (28) est interchangeable et est doté de réacteur électrique autonome raccordé au réseau élec_ trique avec les conducteurs (30) et au générateur (28) et aussi à l'interrupteur (31) général et est logé dans une gaine (26) extractible de quartz optique transpa_
rent placé au contact du liquide descendant pour
l'exposition et la pénétration des rayons avec le
maximum d'énergie en toute direction sur toute  la
masse liquide.

4.Dispositif pour la stérilisation des liquides en géné_
ral, selon la revendication 2, caractérisé en ce que
chaque structure (4) qui forme l'élément (3) et l'élé_
ment (14) est constituée par une enveloppe creuse al_
longée de n'importe quelle forme, dimension et capacité
avec surface (16) intérieure lisse dans laquelle est logé
le générateur (28) de rayons u.v. germicides, la surface
intérieure (16) étant pourvue d'au moins un diaphragme (5)
plat et d'un ou plusieurs diaphragmes (6) avec bague (8)
de canalisation avec courbure (6) vers le haut  ou cour_
bure (7) vers le bas dans le sens contraire au mouvement
du liquide fixés solidairement à la surface (16) placés
de manière transversale et en relation espacée entre eux
pour laisser un intervalle (9) entre la bague (8) et la
surface (29) extérieure de la gaine (26) en déterminant
dans le liquide des passages et des turbulences avec pro_
longement du temps d'exposition du liquide aux rayons u.v.
et pénétration de ceux-ci dans toute la masse liquide.

5.Dispositif pour la stérilisation des liquides en géné_
ral, selon la revendication 2, caractérisé en ce que
il possède un filtre (19) des impuretés en suspension
auto-nettoyant placé à l'entrée du dispositif et est doté
d'un conduit (1) d'entrée, d'éléments (3) et (14) de sté_
rilisation et d'un conduit (22) de descente accouplés et
communiquant entre eux-mêmes dans un circuit ouvert, le
conduit (1), les éléments (3) et (14) et le conduit (22)

0023892

étant de nature à assurer l'équilibre hydrodynamique de l'ensemble avec une quantité de liquide à l'entrée à stériliser égale à la quantité de liquide en sortie sté_ rilisé en l'exposant, tout au long du parcours du cir_ cuit, aux radiations u.v. germicides émises par des gé_ nérateurs spéciaux (28) dans les éléments (3) et (14) de stérilisation dans lesquels des diaphragmes (5), (6) et (7) déterminent des passages forcés et des canalisa_ tions avec variations de l'énergie cinétique du liquide en prolongeant l'exposition aux radiations germicides précedemment citées.

6. Dispositif pour la stérilisation des liquides en général, selon la revendication 2, caractérisé en ce que la struc_ ture (34) en boîtier extérieur de revêtement est pourvue d'un orifice sur une des ses parois latérales pour l'insér tion du conduit (1) d'entrée auquel est accouplée la sou_ pape (20) de réglage de la descente du liquide avec le levier (21) gradué et est doté d'un deuxième orifice sur l'autre paroi latérale pour l'insertion du conduit (22) de descente auquel est raccordé un robinet (42) de prélè_ vement du liquide et d'un ultérieur robinet (37) de dé_ charge du même, et caractérisé en ce que ladite structure (34) est munie d'un volet (35) et d'un couvercle (39) tous deux avec serrure (36) fabriqués à simple ou à double paroi (45) pour délimiter une ou plusieurs chambres her_ métiques pour la préservation de l'humidité des appareils électriques ici logés comprenant un interrupteur (31), un voyant (32) lumineux, un minuteur (33) du temps d'alluma_ ge des générateurs (28) et du fonctionnement du dispositif, et aussi un indicateur de la quantité de liquide stérilisé.

_Fig.1._

_Fig.2._

_Fig.3._

Fig_4_

Fig_6_

Fig_5_

Fig. 7.

Office européen
des brevets

**RAPPORT DE RECHERCHE EUROPEENNE**

0023892

Numéro de la demande

EP 80 83 0053

| Catégorie | Citation du document avec indication, en cas de besoin, des parties pertinentes | Revendication concernée | CLASSEMENT DE LA DEMANDE (Int. Cl. 3) |
|---|---|---|---|
| | **DOCUMENTS CONSIDERES COMME PERTINENTS** | | |
| X | FR - E - 80 549 (G.J.M.A. FEUILLET) <br> * En entier * <br> -- | 1-6 | A 61 L 2/10 <br> C 02 F 1/32 |
| | DE - A - 2 551 622 (R. WIEST) <br> * Figure * <br> -- | 1-6 | |
| | DE - A - 1 916 540 (H. KELLER et al.) <br> * En entier * <br> -- | 1-4 | |
| | CH - A - 330 145 (H. KELLER) <br> * En entier * <br> -- | 1-4 | **DOMAINES TECHNIQUES RECHERCHES (Int. Cl. 3)** <br> A 61 L 2/10 <br> C 02 F 1/32 |
| | US - A - 3 923 663 (W.P. REID) <br> * Figure 1 * -- | 1-6 | |
| A | US - A - 3 061 721 (A. BRENNER) | | |
| A | DE - C - 888 446 (SIEMENS-SCHUCKERT) | | |
| A | DE - A - 2 622 637 (F. BOHNEN-SIEKER) | | **CATEGORIE DES DOCUMENTS CITES** |
| A | GB - A - 1 167 578 (ULTRA DYNA-MICS) | | X: particulièrement pertinent <br> A: arrière-plan technologique <br> O: divulgation non-écrite <br> P: document intercalaire <br> T: théorie ou principe à la base de l'invention <br> E: demande faisant interférence <br> D: document cité dans la demande <br> L: document cité pour d'autres raisons |
| A | FR - A - 413 233 (V. HENRI et al.) | | |
| A | US - A - 3 672 823 (R.M.G. BOUCHER) | | |
| A | US - A - 3 182 193 (S. ELLNER et al.) <br> ---- | | &: membre de la même famille, document correspondant |

Le présent rapport de recherche a été établi pour toutes les revendications

| Lieu de la recherche | Date d'achèvement de la recherche | Examinateur |
|---|---|---|
| La Haye | 12-11-1980 | V. AKOLEYEN |